# EUROPÄISCHE PATENTSCHRIFT

(11) **EP 2 591 824 B1**
(45) Veröffentlichungstag und Bekanntmachung des Hinweises auf die Patenterteilung: **01.04.2020**
(21) Anmeldenummer: 11405366.3
(22) Anmeldetag: 12.12.2011
(51) Int. Cl.: A61Q 5/02, A61Q 19/10, A61K 8/92, A61K 8/97

(54) **Nachhaltige kosmetische und dermatologische Zubereitungen**
Sustainable cosmetic and dermatological formulations
Préparations cosmétiques et dermatologiques durables

(30) Priorität: 13.11.2011 CH 18062011
(43) Veröffentlichungstag der Anmeldung: 15.05.2013
(73) Patentinhaber: Don't Steal My Innovation GmbH, 6015 Luzern (CH)
(72) Erfinder:
(74) Vertreter: Maucher Jenkins Patent- und Rechtsanwälte

(56) Entgegenhaltungen:
- EP-A2- 1 407 756
- WO-A1-01/83665
- WO-A1-2005/121291
- WO-A1-2010/060729
- FR-A1- 2 954 778
- FR-A1- 2 959 753
- JP-A- H11 246 348
- JP-A- H11 255 621
- US-A- 3 083 143
- US-A- 4 035 478
- US-A1- 2005 184 273
- US-A1- 2007 238 780
- US-A1- 2011 124 544
- VISHWANATH PATIL ET AL: "Fatty acid composition of 12 microalgae for possible usein aquaculture feed", INTERNET CITATION, 27. Juni 2006 (2006-06-27), XP002688544, Gefunden im Internet: URL:http://moritz.botany.ut.ee/~olli/b/Pat il07.pdf [gefunden am 2012-01-02]
- DAIO A ET AL: "Manufacture of transparent gel-like soap used as toilet soap, involves mixing olive oil, palm oil, castor oil, coconut oil, water and potassium hydroxide, followed by performing saponification, adding ethanol, heating and cooling", WPI / THOMSON,, Bd. 2009, Nr. 3, 24. Dezember 2008 (2008-12-24), XP002585097, -& JP 2009 185139 A (DAIO ASUKA; HAGIWARA KIYOHIRO; NIKAWA SHOTA; MASUYAMA KAZUTERU; MINOWA) 20. August 2009 (2009-08-20)
- DATABASE WPI Week 199952 Thomson Scientific, London, GB; AN 1999-604888 XP002697759, -& JP H11 263714 A (SHISEIDO CO LTD) 28. September 1999 (1999-09-28) & JP H11 263714 A (SHISEIDO CO LTD) 28. September 1999 (1999-09-28)
- Summer Bee Meadow, Baldwinsville, NY: "soapmaking knowledge and supplies for handcrafted soap maker- Properties of the soapmaking oils", , 14 April 2011 (2011-04-14), Retrieved from the Internet: URL:http://web.archive.org/web/20110414195 414/http://summerbeemeadow.com/content/pro perties-soapmaking-oils [retrieved on 2016-04-29]
- Christina L. Burnett et al.: "Draft amended Final report of CAPB as used in Cosmetics",International journal of toxicology", 2010 Cosmetic Ingredient Review , 18 November 2010 (2010-11-18), pages 31-91, DOI: 10.1177/1091581812447202 Retrieved from the Internet: URL:www.cir-safety.org/sites/default/files /117_final_capb.pdf [retrieved on 2017-05-31]
- Susan Barclay-Nichols: "PEG-7 olivate (or olive oil PEG-7 esters)", , 28 August 2010 (2010-08-28), Retrieved from the Internet: URL:http://swiftcraftymonkey.blogspot.nl/2 010/08/esters-peg-7-olivate-or-olive-oil-p eg-7.html [retrieved on 2017-05-31]
- "Soap making essentials _ Hazelnut oil", , 17 May 2012 (2012-05-17), Retrieved from the Internet: URL:http://web.archive.org/web/20120517170 917/http://www.soap-making-essentials.com/ hazelnut-oil.html [retrieved on 2017-05-31]
- "Resplanta olea", , January 2010 (2010-01), Retrieved from the Internet: URL:https://www.thesoapkitchen.co.uk/acata log/PDF-Resplanta_Olea_Specification.pdf [retrieved on 2017-05-31]
- "Resplanta PGF olea", Res pharma , June 2011 (2011-06), Retrieved from the Internet: URL:http://www.coder-1990.it/1/upload/resp lanta_pgf_tds.pdf [retrieved on 2017-05-31]
- Ecor-Detergente intimo con tree oil , 8 April 2009 (2009-04-08), Retrieved from the Internet: URL:http://forum.saicosatispalmi.org/forum /viewtopic.php?t=8553 [retrieved on 2017-05-31]

## Beschreibung

### Gegenstand und/oder technisches Gebiet

Die Erfindung, die in den Ansprüchen definiert ist, betrifft die Verwendung von wässrigen flüssigen Tensidmischungen (für nachhaltige kosmetische und dermatologische Zubereitungen) als Pflege- oder Reinigungszubereitungen ("Mittel") in Form von Rinse-Off-Formulierungen für Körper und Haare, die sich durch einen aussergewöhnlich hohen Anteil an ungesättigten, langkettigen (>C18) Kohlenwasserstoffresten (Alkylresten) auszeichnen und auf Pflanzenölen der gemässigten Zone basieren.

### Stand der Technik

Haut und Haare werden heute in vielfältiger Weise mit kosmetischen Zubereitungen behandelt. Dazu gehören etwa die Reinigung und Pflege der Haut mit Gesichtswasser, Duschzubereitungen, kosmetische Lotionen, Cremes, Masken, Make-up; die Reinigung der Haare mit Shampoo, die Pflege und Regeneration von Haar und Kopfhaut mit Spülungen und Kuren sowie das Bleichen, Färben und Verformen der Haare mit Färbemitteln, Tönungsmitteln, Wellmitteln und Stylingpräparaten.

Haut und Haare sind täglichen Beanspruchungen, wie beispielsweise, häufiger Reinigung, UV-Licht und Umweltbelastungen und - im Falle des Haares - gelegentlichem Färben oder Dauerwellen ausgesetzt. Insbesondere bei der Körperreinigung führt ein gesteigertes Hygienebewusstsein zu einer immer häufigeren Reinigung. Um hierbei neben optimaler Reinigung und Pflege langzeitverträglich zu sein, müssen die verwendeten Mittel besonders mild zu Haar und zur Haut sein. Dies ist heutzutage nicht immer der Fall - häufig werden Hautirritationen durch Kontakt mit kosmetischen Inhaltsstoffen, wie beispielsweise Tensiden, über einen längeren Zeitraum hinweg ausgelöst. Insbesondere die aufgrund guter Reinigungsleistung und erschwinglichen Preisen breit eingesetzten anionischen Tenside wie Sulfate und deren Ethersulfate, sind laut Literatur als erheblich irritierend zu bezeichnen.^{1,2,3}
¹ Novak, E., Francon, S.T. (1984) : Inflammatory Response to Sodium Lauryl Sulfate in Aqueous Solutions Applied to the Skin of Human Volunteers, Contact Dermatitis, Band 10, S. 101-104.
² Sugar, M., Schmukker, R. (2001): Reduzierung der Hautadsorption von Sodium Laureth Sulfate, SÖfW Journal, Band 127, S.3-5.
³ Effendy, I., Maibach, H.I. (1995): Surfactants and experimental irritant contact dermatitis, Contact Dermatitis, Band 33, S. 217-225.
Ein weiteres Beispiel einer breit eingesetzten Substanzklasse in kosmetischen Zubereitungen sind Polyethylenglycol (PEG) und Alkoxylate, die als Tenside und Emulgatoren eingesetzt werden. In der Literatur werden gegen diese Inhaltsstoffe Bedenken geäussert, da sie Verunreinigungen, wie zum Beispiel 1,4-Dioxan oder Ethylenoxid aus ihrem Produktionsprozess enthalten könnten, welche als Kanzerogene beschrieben werden. Des Weiteren wird vermutet, dass PEG und deren Derivate die Funktion der Hautbarriere herabsetzen und somit anderen Stoffen mit Irritationspotential die Penetration durch die Haut ermöglichen.
Aus WO2005/121291 sind Shampoo-Formulierungen auf Basis von Reaktionsgemischen mit Tensiden aus Fettalkohol oder -amin, z.B. Oleylalkohol, in Kombination mit Kokosdiethanolamid bekannt. Diese Tensidmischung ist kationisch und kann nur bei einem pH unter 5 stabil formuliert werden. Damit kann sie auch nicht als kosmetische oder dermatologische Zubereitung verwendet werden, ohne Hautirritation in Kauf zu nehmen.
Zwar sind an sich milde Tenside aus den unterschiedlichsten Gruppen bekannt, doch befriedigen auch diese zum heutigen Zeitpunkt die Verbraucherwünsche nicht in ausreichendem Masse.
Zunehmend werden von Konsumenten Forderungen laut, dass Kosmetika und deren Inhaltsstoffe gemäss dem Prinzip der Nachhaltigkeit hergestellt werden sollten. Seit mehreren Jahrzehnten gibt es daher Bestrebungen, kosmetische Zubereitungen auf Basis von nachwachsenden Rohstoffen herzustellen, um so das gewünschte Nachhaltigkeitskonzept für diese Konsumgüter zu etablieren.
Dennoch wird heute der Hauptbedarf an Tensiden für Wasch, Pflege- und Reinigungsmittel zu ca. 80% aus fossilen Rohstoffen, d.h. überwiegend aus Erdöl gedeckt. Dies stellt aufgrund der nachteiligen Auswirkungen auf das Klima, möglicher Havarien beim Transport, der Begrenztheit dieser Rohstoffe sowie der politischen Situation der Förderländer, zunehmend eine ökologische, ökonomische und auch politische Herausforderung dar.⁴ Derzeitige Formulierungskonzepte, die auf die Verwendung fossiler Rohstoffe verzichten, beruhen im Wesentlichen auf Palm- und Kokosölen und machen zum heutigen Zeitpunkt lediglich ca. 20% der verwendeten Tenside im Wasch-, Pflege und Reinigungsbereich aus.⁵
⁴ Projektgruppe Nachwachsende Rohstoff (2010: Aktionstag - FORUM WASCHE vom 07. 07.2010
⁵ IKW (2010): Statuspapier zum Einsatz von Palm(kern)öl in WPR Produkten, 29.11.2010
Zunehmend wird jedoch die Nachhaltigkeit dieser Tropenöle in Frage gestellt. Befürchtet wird, dass bei weiterhin steigender Nachfrage nach Palmöl wertvolle Tropenwälder in den Produktionsländern dem intensiven Anbau von Ölpalmen zum Opfer fallen. Zusätzlich werden solche Plantagen unter massivem Einsatz von Herbiziden und Düngemittel betrieben. Um eine nachhaltige Produktion und Nutzung von Palmöl zu fördern, hat der Roundtable on Sustainable Palmoil (RSPO) Richtlinien verabschiedet, welche die Erfüllung sozialer und ökologischer Mindestbedingungen vorschreiben. Dieses System wird von NGOs und anderen Organisationen jedoch kontrovers diskutiert,⁶ da Zertifizierungssysteme die grundsätzlichen Probleme nicht zu lösen vermögen. Zusätzlich zu den genannten Herausforderungen in den Anbauregionen, wie Indonesien, Philippinen und Malaysia, tragen die weiten Transportwege zu den Hauptabnehmermärkten, welche sich vorwiegend in Europa und Nordamerika befinden, zu einer Erhöhung des CO₂-Gehalts in der Atmosphäre bei.
*⁶* WWF Deutschland (2007): Regenwald für Biodiesel? Ökologische Auswirkungen der energetischen Nutzung von Palmöl

Wünschenswert für die Schonung der natürlichen Ressourcen und als Beitrag für den weltweiten Klimaschutz, ist daher die Verwendung von lokalen und/oder regionalen Ölen aus Anbaugebieten der gemässigten Zonen, wie z.B. aus dem geographischen Europa. Die Folge eines solchen Rohstoffkonzeptes für kosmetische Zubereitungen wäre ein verstärkter landwirtschaftlicher Anbau von industriellen Ölsaaten in den gemässigten Zonen, verbunden mit einem positiven Einfluss auf Beschäftigungsgrad, Handelsbilanz, Biodiversität, sowie Gesundheit und Umwelt.

Es wäre somit aus ökologischen, ökonomischen, sowie soziopolitischen Gründen vorteilhaft, kosmetische Zubereitungen herzustellen, welche auf pflanzlichen, erneuerbaren Rohstoffen beruhen und weder fossile, noch palm(kern)- oder kokosölbasierende Inhaltsstoffe enthalten.

Im Gegensatz zu der Verseifung von Pflanzenölen zur Herstellung von Seifenstücken, sowie der Verwendung von tierischen Fetten für Waschzwecke, ist eine Kombination von kosmetischen Inhaltsstoffen, insbesondere von Tensiden, die ausschliesslich auf Pflanzenölen der gemässigten Zonen basieren, aufgrund technischer Einschränkungen bisher nicht verfügbar.
So weisen beispielsweise die in Betracht kommenden Fettsäuregemische aus Pflanzen der gemässigten Zonen in heute üblichen Formulierungskonzepten für Wasch- und Reinigungsanwendungen eine schlechtere Wasserlöslichkeit und schlechtere Reinigungskraft auf. Dies liegt hauptsächlich an dem hohen Anteil an langkettigen Fettsäuren (> C18). Als Folge sind die mit Abstand am häufigsten in Dusch- und Reinigungszubereitungen verwendete anionischen Tenside C12-Verbindungen wie Laurylbenzolsulfonate, Laurylethersulfate, oder Laurylsulfat auf Basis von Palm- , Kokos- oder Erdöl.
Weiterhin stellen Zubereitungen aus pflanzlichen Rohstoffen der gemässigten Zonen - nicht zuletzt durch ihren grossen Anteil an ungesättigten Fettsäuren, die leicht oxidieren - hohe Anforderungen an die Stabilität der Formulierung. So kann es zum Beispiel in Lotionen, Cremes, oder wässrigen Tensidmischungen durch die ungesättigten Fettsäurederivate zu unerwünschten Verfärbungen, ranzigem Geruch und/oder Phasentrennungen kommen. Aufgrund dieser Limitierungen, wurde der Ersatz von tropischen C12/C14 Fettsäuren durch Europäische Fettsäuren mit C> 18 bisher als unwahrscheinlich eingeschätzt.⁷
*⁷* Claude, Sylvain (1999) : Oleochemistry as an opportunity to consolidate the sustainable development of European oil crops - an updated prospect, Oléagineux, Corps Gras, Lipides, Band 6, Heft 5, S. 418-27*.*

Die komplexe Aufgabe der Erfindung hat somit darin bestanden, kosmetische Zubereitungen mit Inhaltsstoffen, wie zum Beispiel Tensiden zu formulieren, die auf pflanzlichen Rohstoffen der gemässigten Zonen beruhen um einerseits die Transportwege der Rohstoffe zu verkürzen und andererseits die negativen Auswirkungen bei der Gewinnung von Palmöl oder durch den Einsatz von Erdöl zu reduzieren. Anbau und Produktion der Rohstoffe sollten dabei soweit als möglich regional erfolgen. Folglich galt es, stabile Mittel herzustellen, die einen überwiegenden Anteil an kosmetischen Inhaltsstoffen mit Kohlenwasserstoffkettenlängen (Alkylkettenlängen) von C>18 beinhalten und mehrheitlich ungesättigt sind, wie sie primär in Pflanzenölen der gemässigten Zonen vorkommen.

Überraschenderweise wurde nun ein Weg gefunden, kosmetische Zubereitungen rein auf Basis von Pflanzenölen der gemässigten Zonen und mit einem überwiegenden Anteil an Tensiden abgeleitet von Kohlenwasserstoffketten (Alkylketten) mit C>18 und mehrheitlich ungesättigt, stabil zu formulieren.
Durch eine gezielte neuartige Auswahl der kosmetischen Inhaltsstoffe, wie beispielsweise der Tenside in Bezug auf Kohlenwasserstoffkettenlängen (Alkylkettenlängen) und Sättigungsgrad, sowie Kombination mit Hilfsmitteln zur Stabilisierung der Formulierung, wurden entgegen der Erwartungen stabile Zubereitungen mit hoher Reinigungs- und Pflegeleistung hergestellt.

Diese Erfindung erlaubt somit erstmalig auf Basis von pflanzlichen Ölen als nachwachsende Rohstoffe aus den gemässigten Zonen umweltschonende und nachhaltige kosmetische Zubereitungen herzustellen.

Gegenstand dieser Erfindung, wie in den Ansprüchen konkret definiert, ist somit die Verwendung von Tensidmischungen für neue kosmetische oder dermatologische Zubereitungen als Reinigungs- und/oder Pflegemittel, die sich durch einen aussergewöhnlich hohen Anteil an ungesättigten, langkettigen (>C18) Kohlenwasserstoffresten (Alkylresten) auszeichnen und auf Pflanzenölen der gemässigten Zone basieren.

Die erfindungsgemässe Verwendung kann in Form von wässrigen Tensidzubereitungen erfolgen, wie Dusch- und Badezubereitungen, flüssigen Seifen und Syndets, Reinigungsgelen oder-lotionen, Shampoos, Mitteln zur Körper-, Haar- und Gesichtsreinigung, speziellen Reinigungsmitteln für Kinder sowie für empfindliche Körperpartien wie den Augenbereich oder für Personen mit empfindlicher oder irritierter Haut, welche allenfalls zu allergischen Reaktionen neigt.

Im Rahmen der vorliegenden Erfindung stehen gemässigte Zonen als diejenigen Klimazonen, welche geographisch zwischen den Subtropen und der kalten Zone gelegen sind, wie zum Beispiel das geographische Europa.

Die im Rahmen der erfindungsgemässen Lehre genannten Pflanzenöle der gemässigten Zone umfassen Pflanzenöle, -wachse,- fette oder -harze, die aus Pflanzen gewonnen werden, die entweder ursprünglich aus den gemässigten Zonen stammen, oder von Pflanzen stammen, die in den gemässigten Zonen angebaut werden oder gedeihen können.

Bevorzugt handelt es sich hierbei um natürliche Triglyceride mit einer Fettsäurenverteilung von Fettsäuren mit Kohlenwasserstoffketten (Alkylketten) von 18 und mehr Kohlenstoffatomen von über 60 Gew.-%, besonders bevorzugt über 70 Gew.-% und ganz besonders bevorzugt von über 80 Gew.-%, sowie von Fettsäuren mit Kohlenwasserstoffketten (Alkylketten) von 16 und weniger Kohlenstoffatomen von unter 20 Gew.-%, insbesondere von Kohlenwasserstoffketten (Alkylketten) mit 14 C-Atomen und weniger von unter 5 Gew-%, wobei der Anteil an ungesättigten Fettsäuren in diesen Triglyceriden über 50 Gew.-%, vorzugsweise über 60 Gew.-% und besonders bevorzugt zwischen 70 und 90 Gew.-% liegt, bezogen auf den Gesamtgehalt an Fettsäuren im Pflanzenöl.

Im Sinne dieser Anmeldung stehen Inhaltsstoffe auf Basis von Pflanzenölen, bzw. Inhaltsstoffe, die von Ölen, Wachsen, Fetten oder Harzen abgeleitet sind - soweit nicht anders angegeben - für die funktionelle Gruppe umfassend Fette und Öle, Ölkörper, Emulgatoren und Tenside mit lipophilen (fettlöslichen) Teilen. Diese Inhaltsstoffe erfüllen zum Teil weitere Funktionen in der Zubereitung, wie zum Beispiel als Verdickungsmittel, Feuchtigkeitsmittel, Dispergiermittel, Filmformer, Konditionierungsmittel und andere.

Im Rahmen der vorliegenden Erfindung steht - soweit nicht anders angegeben - auf Basis von Pflanzenölen stellvertretend für Derivate aus Fettsäuren - gereinigt oder als Gemisch -, und/oder deren Reaktionsprodukte, wie beispielsweise Fettalkohole und deren Ether und Ester, Amine oder Fettsäureamide, Fettsäureester, Hydrierungsprodukte, Produkte von Additionsreaktionen an den Doppelbindungen.

Soweit nicht explizit anders angegeben, steht im Rahmen der vorliegenden Erfindung Alkyl- und Acyl- für unverzweigte, gesättigte oder ungesättigte Reste.

Stoffe, die als Inhaltsstoffe von kosmetischen Mitteln dienen, werden nachfolgend gemäss der International Nomenclature Cosmetic Ingredient- (INCI-) Nomenklatur bezeichnet. Die INCI-Bezeichnungen sind dem "International Cosmetic Ingredient Dictionary and Handbook, 13th Edition (2010)" zu entnehmen. Herausgeber: The Personal Care Products Council.

Soweit nicht explizit anders angegeben, beziehen sich die angegeben Menge in Gewichtsprozent (Gew.-%) auf die gesamte Zubereitungen. Dabei beziehen sich die prozentualen Mengenangaben auf Aktivgehalte.

### Beschreibung

### Beschreibung der kosmetischen Zubereitung

Im Sinne dieser Anmeldung, wie in den Ansprüchen definiert, enthält das, als kosmetische Pflege- oder Reinigungszubereitung vorgesehene Mittel, ein oder mehrere erfindungsgemäss verwendete Tenside mit lipophilen (fettlöslichen) Teilen, die sich dadurch kennzeichnen, dass die Tenside einen überwiegenden Anteil an Kohlenwasserstoffkettenlängen (Alkylkettenlängen) von 18 und mehr C-Atomen tragen und mehrheitlich ungesättigt sind.

Für die erfindungsgemässe Verwendung geeignete Tenside sind anionische, nichtionische und/oder zwitterionische Tenside, welche in dieser Anmeldung im Abschnitt "Tenside" detailliert beschrieben werden.

Die Zubereitung enthält bei der erfindungsgemässen Verwendung vorzugsweise ein oder mehrere Tenside, welche von Pflanzenölen abgeleitet sind, und ausgewählt sind aus der Gruppe:
a) Fettsäuren und deren Alkali oder Ammoniumsalze mit den allgemeinen Formeln R(O)OH bzw. R(O)OM mit M = Alkalimetallkation oder Ammonium, beispielsweise anionische Tenside wie Seifen aus Alkali- oder Ammoniumsalz(e) von Fettsäuren (Seifen) und andere
b) Fettsäureester mit der allgemeinen Formel R(O)OR', beispielsweise Acylisethionat, Acyllactylat, sulfonierte Fettsäuren, sulfonierte Fettsäureester, wie sulfonierte Fettsäureglycerinester und sulfonierte Fettsäuremethylester, Polyhydroxyfettsäureester, sowie Glyceryl- und Polyglycerylester mit 1-20 Glycerineinheiten, Carbonsäureester, Sorbitanester, und andere
c) Ether von Fettalkoholen mit der allgemeinen Formel ROR', beispielsweise ethoxylierte und propoxylierte Fettalkohole, Polyoxyethylenglycole, Glykolipide, wie zum Beispiel Alkylpolyglykoside mit 1-10 Glykosideinheiten, Alkylsulfate und Alkylethersulfate, Alkylphosphate und Alkyletherphosphate, Sulfosuccinatester und andere
d) Fettsäureamide mit der allgemeinen Formel R(O)NR', beispielsweise N-Acylaminosäurederivate, wie beispielsweise N-Acylaspartat, N-Acylglycinat, N-Acylalaninat, N-Acylsarkosinat oder N-Acylglutamat, acylierte Polypeptiden, N-Acylaminosulfonsäuren, wie N-Acyltaurid, Carbonsäureamidethersulfate, Alkanolamin-Carbonsäure-Kondensate, Amidoalkylpyrrolidone, Amidoamine und Aminobetaine, acylierte Diamine und Polyamine, sowie deren Salze und andere
e) Fettsäureimine der allgemeinen Formel R(NR')NR"R"', beispielsweise Imidazolcarboxylate, bzw. Amphoacetate und andere
f) Primäre, sekundäre, tertiäre oder quartäre Fettamine bzw. Fettammoniumsalze der allgemeinen Formeln RNH2, RHNR', RNR'R" und (R)(R')(R")(R"')N+X-, beispielsweise N-Alkylaminopropionsäure, N-Alkyliminodipropionsäure, N-Alkylbetaine, Sulfobetaine, bzw. Sultaine, Polyoxyethylen (POE) amine, quaternisierte POE amine, Aminoxide, Triethanolaminester quats (TEAEQ), imidazolinium und dialkyldimethyl ammonium ester quats und andere,
g) sowie Gemische derselben
   i) wobei R' bis R'" organische oder anorganische hydrophile Reste darstellen und X- für ein Anion steht
   ii) wobei R den lipophilen Teil des Tensids darstellt und aus einem linearen, gesättigten oder ungesättigten, unverzweigten Kohlenwasserstoffrest (Alkylrest) mit 8 bis 24 Kohlenstoffatomen besteht und der Anteil an Kohlenwasserstoffketten (Alkylketten) von 18 und mehr C-Atomen über 60 Gew.-%, vorzugsweise über 70 Gew.-% und besonders bevorzugt zwischen 80-100 Gew.-% beträgt, jeweils bezogen auf den Gesamtgehalt an Kohlenwasserstoffketten (Alkylketten) R der im Mittel enthaltenen Tenside.

Gleichzeitig beträgt der Anteil an einfach und/oder mehrfach ungesättigten Kohlenwasserstoffketten (Alkylketten) R der Tenside bei deren erfindungsgemässer Verwendung in dem Mittel 50-100 Gew.-%, vorzugsweise zwischen 60-100 Gew.-% und besonders bevorzugt zwischen 70-90 Gew.-%, jeweils bezogen auf den Gesamtgehalt an Kohlenwasserstoffketten (Alkylketten) R der im Mittel enthaltenen Tenside.

Des Weiteren kann das Mittel bei der erfindungsgemässen Verwendung zu einem geringeren Teil Tenside mit kürzeren Kohlenwasserstoffketten (Alkylketten) enthalten, so dass der Anteil an kurzen Kohlenwasserstoffkettenlängen (Alkylkettenlängen) in den Tensiden von R <=C16 unter 20 Gew.-%, vorzugsweise unter 15 Gew.-% und insbesondere <= C 14 unter 5 Gew.-% beträgt, jeweils bezogen auf den Gesamtgehalt an Kohlenwasserstoffketten (Alkylketten) R der im Mittel enthaltenen Tenside.

### Pflanzen der gemässigten Zonen

Die kosmetischen Zubereitungen enthalten bei der erfindungsgemässen Verwendung 80-100 Gew.-%, vorzugsweise mehr als 95 Gew.-%, besonders bevorzugt mehr als 99 Gew.-%, äusserst bevorzugt 100 Gew.-% Tenside, die von Pflanzenölen der gemässigten Zonen abgeleitet sind, bezogen auf den Gesamtgehalt an Tensiden.

Bei den Pflanzenölen handelt es sich hierbei um Öle, Wachse, Fette oder Harze aus den bevorzugten folgenden Pflanzenfamilien sowie deren Gemische: Birkengewächse, Doldengewächse, Fuchsschwanzgewächse, Geissblattgewächse, Granatapfelgewächse, Hahnenfussgewächse, Hanfgewächse, Heidekrautgewächse, Hülsenfrüchtler, Johanniskrautgewächse, Kieferngewächse, Korbblütler, Kreuzblütengewächse, Leingewächse, Lippenblütler, Lorbeergewächse, Malvengewächse, Mohngewächse, Nachtkerzengewächse, Nachtschattengewächse, Ölbaumgewächse, Ölweidengewächse, Raublattgewächse, Rosengewächse, Sauergrässer, Sapotengewächse, Sesamgewächse, Silberbaumgewächse, Simmondsiaceae, Stachelbeergewächse, Sumachgewächse, Süssgrässer, Teestrauchgewächse, Walnussgewächse, Weinrebengewächse und Wolfsmilchgewächse.

Erfindungsgemäss genutzte Vertreter sind hierbei Amarant, Anis, Apfel, Aprikose, Arnika, Avocado, Baumwolle, Borretsch, Brokkoli, Hanf, Haselnuss, Buche, Buchsbaum, Distel, Dinkel, Erdnuss, Erdmandel, Flieder, Gartenkresse, Gerste, Granatapfel, Hafer, Heidelbeere, Holunder, Jasmin, Jatropha, Johannisbeere, Johanniskraut, Jojoba, Kamelie, Kamille, Kümmel, Kirsche, Karotte, Koriander, Königskerzen, Krambe, Kreuzblättrige Wolfsmilch, Kürbis, Iberischer Drachenkopf, Lavendel, Leindotter, Leinsamen, Liguster, Lupine, Luzerne, Mais, Mandel, Mirabelle, Mango, Mohn, Nachtkerze, Olive, Ölrettich, Ölrauke, Pekannuss, Pfirsich, Pflaume, Pistazien, Preiselbeere, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut undWildrose und Gemische derselben.

Vorzugsweise ist das Öl ausgewählt aus der Gruppe: Brokkoli, Hanf, Haselnuss, Buche, Distel, Dinkel, Erdmandel, Gerste, Kirsche, Königskerzen, Krambe, Kreuzblättrige Wolfsmilch, Kürbis, Iberischer Drachenkopf, Leindotter, Leinsamen, Lupine, Luzerne, Mais, Mohn, Nachtkerze, Olive, Ölrettich, Ölrauke, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube und Weizen und Gemische derselben.

Die Zubereitungen basieren bei der erfindungsgemässen Verwendung auf mindestens einem Tensid auf Basis von Pflanzenölen der gemässigten Zonen. Hierbei handelt es sich um Tenside, welche wiederum auf einem oder der Kombination mehrerer Pflanzenöle der gemässigten Zone basieren.

### Beschreibung der Tenside

Im Sinne dieser Anmeldung enthalten die Zubereitungen bei der erfindungsgemässen Verwendung vorzugsweise eine oder mehrere Klassen an Tensiden, die auf einer Mischung von Kohlenwasserstoffketten (Alkylketten) unterschiedlicher Kettenlänge (C6-C24) und Sättigungsgraden (gesättigt, einfach ungesättigt, mehrfach ungesättigt) beruhen. Hierbei entspricht die Verteilung an Kohlenwasserstoffketten (Alkylketten) in Bezug auf Kettenlänge und Sättigungsgrad dem natürlichen Vorkommen im Pflanzenöl.

Indem für die Synthese der Tenside native Fettsäuren- oder Fettsäureestergemische verwendet werden - wie sie bei der Verseifung oder Alkoholyse der natürlich vorkommenden Pflanzenöle oder -fette anfallen - können die Tenside kostengünstig, ressourceneffizient und umweltschonend produziert werden. Zusätzlich zu den ökologischen und ökonomischen Vorteilen durch die Verwendung von natürlichen Fettsäuregemischen, zeigten die verwendeten kosmetischen Inhaltstoffe auf Basis von Fettsäuregemischen technische Vorzüge, wie eine verbesserte Stabilität der erfinderischen Zubereitungen.

Hierbei kann ein Teil des entstehenden Glyzerins in dem Produktgemisch verbleiben, so dass des Weiteren auf aufwändige Reinigungsprozesse verzichtet werden kann. Der Anteil an Glycerin in dem Tensidgemisch beträgt maximal 15 Gew.-%, bevorzugt unter 13 Gew.-% und ganz besonders bevorzugt unter 11 Gew.-%, bezogen auf den Gesamtgehalt an Tensiden.

### Tenside

Als oberflächenaktive Inhaltsstoffe auf Basis von Pflanzenölen eignen sich für die Zubereitungen bei der erfindungsgemässen Verwendung Tenside aus den Klassen der anionischen, nichtionischen, kationischen, zwitterionische und/oder amphoteren Tenside, wobei der lipophile Teil der Tenside vorzugsweise auf Basis von Pflanzenölen der gemässigten Zonen beruht, insbesondere auf Basis von natürlichen Fettsäuregemischen, wie sie in Pflanzenölen auftreten.
Aus anwendungstechnischer Sicht werden erfindungsgemäss anionische, nichtionischen, sowie zwitterionische und/oder amphotere Tenside, sowie deren Kombinationen, eingesetzt.

Geeignete anionische Tenside sind beispielsweise Seifen aus Alkali- oder Ammoniumsalz(e) von Fettsäuren (Seifen), Alkylbenzolsulfonate, Alkan-/Alkensulfonate, Alkylsulfate bzw. Fettalkoholsulfate, Alkylpolyglykolethersulfate mit 2 bis 6 Ethylenoxideinheiten (EO) im Etherteil, sowie Sulfosuccinate, Sulfobernsteinsäuremono- und di-Alkylester, α-Olefinsulfonate, Alkylsulfoacetate, sulfonierte Fettsäuren, sulfonierte Fettsäureester, wie sulfonierte Fettsäureglycerinester und sulfonierte Fettsäuremethylester, Alkylphosphate und Alkyletherphosphate, Phosphor- und Polyphosphorsäureester, Carbonsäureamidethersulfate, N-Acylaminosäurederivate, wie beispielsweise N-Acylaspartat, N-Acylglycinat, N-Acylalaninat, N-Acylsarkosinat oder N-Acylglutamat, acylierte Polypeptide, N-Acylaminosulfonsäuren, wie N-Acyltaurid, Alkylisethionat, Acylisethionat, Acyllactylat, Fettalkoholcarboxylat, sowie Alkylpolyglykolethercarboxylat bzw. deren Mischungen. Die anionischen Tenside können in Form ihrer Alkali-, Erdalkali- oder Ammoniumsalze, sowie als lösliche Salze organischer Basen, wie Mono-, Di- oder Tri- bzw. Tetraalkylammonium vorliegen, sowie in Form ihrer korrespondierenden Säure.

Vorzugsweise werden in den Zubereitungen erfindungsgemäss anionische Tenside verwendet, deren hydrophiler Teil aus pflanzlichem Ursprung stammt, besonders bevorzugt abgeleitet von Pflanzen der gemässigten Zone, wie beispielsweise N-Acylaminosäurederivate, wie beispielsweise N-Acylaspartat, N-Acylglycinat, N-Acylalaninat, N-Acylsarkosinat oder N-Acylglutamat, acylierte Polypeptide, N-Acylaminosulfonsäuren, wie N-Acyltaurid, Alkylisethionat, Acylisethionat, Acyllactylat und Fettalkoholcarboxylat.
Die Zubereitungen werden vorzugsweise alkylsulfat- und alkylethersulfatfrei hergestellt. Der Gehalt an anionischen Tensiden in den Zubereitungen beträgt bei der erfindungsgemässen Verwendung typischerweise zwischen 0 und 40 Gew.-%.

Als nichtionische Tenside eignen sich beispielsweise Alkoholpolyglykolether und Carbonsäurepolyglykolester, d.h. ethoxylierte und/oder propoxylierte Alkohole oder Carbonsäuren mit 1-40 Ethylenoxid (EO) und/oder Propylenoxid (PO)- Einheiten, Aminoxide, Polyethylenglykolmercaptane, Glykolipide, wie zum Beispiel Alkylpolyglykoside mit 1-10 Glykosideinheiten, Polyhydroxyfettsäureamide, Polyhydroxyfettsäureester, sowie Glyceryl- und Polyglycerylester mit 1-20 Glycerineinheiten, Carbonsäureester, Sorbitanester, sowie alkoxylierte Sorbitanester, Alkanolamin-Carbonsäure-Kondensate, N-Alkylpyrrolidone, Amidoalkyl-2-pyrrolidone.

Vorzugsweise werden nichtionische Tenside verwendet, deren hydrophiler Teil natürlichen Ursprungs ist, wie beispielsweise Glykolipide, Polyhydroxyfettsäureester, Glyceryl- und Polyglycerylester, Carbonsäureester oder Sorbitanester.
Der Gehalt an nichtionischen Tensiden in den Zubereitungen beträgt bei der erfindungsgemässen Verwendung typischerweise zwischen 0 und 40 Gew.-%.

Es hat sich weiter überraschend gezeigt, dass die Zubereitungen bei der erfindungsgemässen Verwendung vorzugsweise frei von Polyethylenglykolen und/ oder Alkoxylaten formuliert werden können. Bevorzugte Zubereitungen sind bei der erfindungsgemässen Verwendung daher dadurch gekennzeichnet, dass sie keine Verbindungen der Formel (I) H-(O-CH2CH2)n-OH(I), in der n für ganze Zahlen zwischen 1 und 100.000 steht, enthalten. Durch die Vermeidung dieser Substanzen eignen sich die Zubereitungen bei erfindungsgemässer Verwendung insbesondere für Anwendungen auf empfindlicher oder irritierter Haut und/oder in Kinderprodukten.

Als amphotere und/oder zwitterionische Tenside eignen sich beispielsweise N-Alkylaminopropionsäuren, Alkyl-iminodipropionsäuren, Imidazolcarboxylate, N-Alkylbetaine, Amidoamine und Amidobetaine, Aminoxide, Sulfobetaine und Sultaine. Bei der erfindungsgemässen Verwendung beträgt der Gehalt an amphoteren und/oder zwitterionischen Tensiden in den Zubereitungen typischerweise zwischen 0 und 40 Gew.-%.

Vorzugsweise werden amphotere Tenside verwendet, deren hydrophiler Teil natürlichen Ursprungs ist und nicht von Erdöl abgeleitet ist.

Zusätzlich verwendete geeignete kationische Tenside, welche vornehmlich als Haut-oder Haarkonditionierungsmittel eingesetzt werden, sind beispielsweise primäre, sekundäre, tertiäre oder quartäre Alkylammoniumsalze der Formel (RI)(RII)(RIII)(RIV)N+X-, in der RI bis RVI für vier gleich- oder verschiedenartige Alkylreste und X- für ein Anion stehen.

Neben den hier genannten Inhaltstoffen können bei der erfindungsgemässen Verwendung die Zubereitungen weiterhin auch Tenside enthalten, die auf Basis von Tier- oder petrochemischen Ölen und Fetten beruhen, sowie Silikone, Siloxane, Pyrrolidone.

Der Gehalt an weiteren Tensiden, die nicht auf Basis von Pflanzenölen beruhen, liegt in den Zubereitungen bei der erfindungsgemässen Verwendung bei maximal 20 Gew.-%, vorzugsweise bei weniger als 5 Gew.-%, insbesondere bevorzugt zwischen 0 und 1 Gew.-%, äusserst bevorzugt ist die erdöl- und palmölfreie Ausführungsform, d.h. mit einem Gehalt an weiteren Tensiden bei 0 Gew.-%, jeweils bezogen auf den Gesamtgehalt an Tensiden.

Bevorzugt werden bei der erfindungsgemässen Verwendung die Zubereitungen silikonfrei hergestellt und vertrieben.

In einer weiteren bevorzugten Ausführungsform sind bei der erfindungsgemässen Verwendung die Zubereitungen dadurch gekennzeichnet, dass sie weder Alkylsulfate, Alkylethersulfate, Silikone oder PEG-Derivate enthalten und somit besonders milde kosmetische Zubereitungen hergestellt werden können. Dies findet insbesondere Anwendung für Konsumenten mit empfindlicher oder irritierter Haut, in Naturprodukten und für Haushalte mit besonders schutzbedürftigen Personen (z.B. Kinder, Personen mit psychischen oder physischen Einschränkungen etc.). Ganz besonders eignen sich die erfindungsgemässen Zubereitungen für Dusch- und Bademittel für Kinder, die Ziepen und Tränen bei Augenkontakt (z.B. "no tears Shampoos") vermeiden sollen und für Zubereitungen zur Anwendung im Augenbereich, wie beispielsweise bei Produkten zum Abschminken.

### Weitere Zusatzstoffe & Eigenschaften der kosmetischen Zubereitung

### pH- Stellmittel

Bei der erfindungsgemässen Verwendung kann der pH-Wert der Zubereitung mittels üblicher pH-Regulatoren eingestellt werden, wobei je nach Anwendung dem Fachmann bekannte, unterschiedliche pH-Bereiche von sauer (pH 1-4) zu neutral (pH 5-7) bis hin zu basisch (pH 8-11) eingestellt werden.

### Antioxidantien

Bevorzugt werden bei der erfindungsgemässen Verwendung der kosmetischen Zubereitung Antioxidantien zugesetzt, einerseits um die ungesättigten Kohlenwasserstoffketten (Alkylketten) der Zubereitung zu schützen und andererseits, um bei der Anwendung schädliche oxidative Einflüsse auf Haut bzw. Haare zu mindern. Beispielsweise werden die Antioxidantien ausgewählt aus der Gruppe bestehend aus Aminosäuren (z.B. Glycin, Histidin, Tyrosin, Tryptophan) und deren Derivaten, Imidazole (z.B. Urocaninsäure) und deren Derivaten, Peptide wie D,L-Carnosin, D-Carnosin, L-Carnosin und deren Derivaten (z.B. Anserin), Carotinoide, Carotine und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, [gamma]-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z.B. [alpha]-Hydroxyfettsäuren, Palmitinsäure, Phytinsäure, Lactoferrin), [alpha]-Hydroxysäuren (z.B. Citronensäure, Milchsäure, Apfelsäure), Numinsäure, Gallensäure, Gallenextrakte, Bilirubin, Biliverdin, EDTA, EGTA und deren Derivate, ungesättigte Fettsäuren und deren Derivate (z.B. [gamma]-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, [alpha]-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid), Superoxid-Dismutase und die erfindungsgemäss geeigneten Derivate (Salze, Ester, Ether, Zucker, Nukleotide, Nukleoside, Peptide und Lipide) dieser genannten Stoffe. Erfindungsgemäss bevorzugt sind Antioxidantien, die auf Rohstoffen aus Pflanzen, bevorzugt Pflanzen der gemässigten Zonen beruhen, wie beispielsweise Antioxidantien aus den Gruppen der Aminosäuren, Peptide, Cartinoide, Chelatoren, Pflanzenextrakten und Hydroxysäuren, sowie Mischungen derselben.

Die Menge der Antioxidantien (eine oder mehrere Verbindungen) in den Zubereitungen beträgt bei der erfindungsgemässen Verwendung vorzugsweise 0,001 bis 30 Gew.-%, besonders bevorzugt 0,05-20 Gew.-%, insbesondere 1-10 Gew.-%, bezogen auf das Gesamtgewicht der Zubereitung. Diese können vom Fachmann mit anderen hier genannten Inhaltstoffen kombiniert werden

### UV-Filter

Es ist auch möglich und vorteilhaft im Sinne der vorliegenden Erfindung, einen Gehalt an UV-Schutzsubstanzen zu verwenden. Vorteilhaft können daher bei der erfindungsgemässen Verwendung der Zubereitungen Substanzen enthalten sein, die UV-Strahlung im UVB-Bereich absorbieren, wobei die Gesamtmenge der Filtersubstanzen vorzugsweise zwischen 0,1 Gew.-% bis 30 Gew.-% betragen kann. Bevorzugt sind handelsübliche Wasser- oder öllösliche UVB-Filter. Es kann auch von Vorteil sein, in erfindungsgemässen Zubereitungen UVA-Filter einzusetzen, die üblicherweise in kosmetischen und/oder dermatologischen Zubereitungen enthalten sind. Es können die gleichen Mengen eingesetzt werden, welche für UVB-Filter genannt wurden. Üblicherweise werden Kombinationen von UV-Filtern eingesetzt, die vom Fachmann mit anderen hier genannten Inhaltstoffen kombiniert werden können.

### Konservierungsmittel

Bei der erfindungsgemässen Verwendung kann die Zubereitung alle in Kosmetika üblichen Konservierungsmittel enthalten.

Erfindungsgemäss geeignet sind beispielsweise Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff- und Stickstoff-Acetale sowie -Formale, Benzamidine, Isothiazole und deren Derivate wie Isothiazolinone, Phtalimidderivate, Pyridinderivate, oberflächenaktive Verbindungen, Guanidine, antimikrobielle amphoterer Verbindungen, Chinoline, 1,2-Dibrom-2,4-dicyanobutan, Iodo-2-propynyl-butyl-carbamat, lod, lodophore und Peroxide.

Besonders bevorzugt ist eine milde Konservierung der Zubereitung bei erfindungsgemässer Verwendung auf Basis von Wirkstoffen, ausgewählt aus Ethanol, Benzylalkohol, Dehydroessigsäure und deren Salzen, pflanzlichen organischen Säuren, Glycerin, Zitronensäure, Milchsäure, Benzoesäure, Salicylsäure, vorzugsweise in Kombination mit einem der erfindungsgemäss bevorzugten Komplexbildner.

Die hier genannten Inhaltstoffe können vom Fachmann mit anderen kosmetischen Inhaltstoffen kombiniert werden.

### Farb- und Duftstoffe

Bei der erfindungsgemässen Verwendung können die Zubereitungen auch Farbstoffe zur direkten oder oxidativen Färbung von Haaren enthalten. Die Färbezubereitungen sollen das Haar schützen und möglichst wenig schädigen. Auf Basis der erfindungsgemässen Zubereitungen können sowohl Haarfärbemittel als auch Tönungsshampoos hergestellt werden.

Um den ästhetischen Eindruck der Zubereitungen zu verbessern, können bei der erfindungsgemässen Verwendung alle in Kosmetika üblichen Duft- und Farbstoffe zugesetzt werden.
Bevorzugte Farbstoffe und Duftstoffe, deren Auswahl dem Fachmann keinerlei Schwierigkeit bereitet, besitzen eine hohe Lagerstabilität und Unempfindlichkeit gegenüber den übrigen Inhaltsstoffen der kosmetischen Zubereitung.
Besonders bevorzugt werden Farbstoffe und Duftstoffe, welche in der Literatur nicht als Allergene beschrieben werden und auf natürlichen Rohstoffen basieren, wie zum Beispiel Extrakte aus Pflanzen, vorzugsweise aus den gemässigten Zonen.

In einer weiteren bevorzugten Ausführungsform der Erfindung, werden weder Farb-, noch Duftstoffe zugesetzt, beispielsweise für Anwendungen bei Konsumenten mit Allergien und/oder sensibler Haut.

### Viskosität

Bei der erfindungsgemässen Verwendung kann die Zubereitung alle in der Kosmetik üblichen, dem Fachmann bekannten, Viskositätsregulatoren enthalten und kann mit anderen kosmetischen Inhaltsstoffen in der erfindungsgemäss verwendetenZubereitung kombiniert werden. Die Viskosität wird je nach Anwendung auf die dem Fachmann bekannten und im Markt üblichen Werte eingestellt.
Geeignete Viskositätsregulatoren sind beispielsweise organische abgewandelte Naturstoffe (Carboxymethylcellulose und andere Celluloseether, Hydroxyethyl- und - propylcellulose und dergleichen, Kernmehlether), organische vollsynthetische Verdickungsmittel (Polyacryl- und Polymethacryl-Verbindungen, Vinylpolymere, Polycarbonsäuren, Polyether, Polyimine, Polyamide) und anorganische Verdickungsmittel (Polykieselsäuren, Schichtsilikate, Tonmineralien wie Montmorillonite, Zeolithe, Kieselsäuren), sowie organische natürliche Verdickungsmittel (Agar-Agar, Carrageen, Xanthan, Traganth, Gummi arabicum, Alginate, Pektine, Polyosen, GuarMehl, Johannisbrotbaumkernmehl, Stärke, Dextrine, Gelatine, Casein) und Mischungen derselben.

Bevorzugte Viskositätsregulatoren sind natürliche organische Verdickungsmittel aus pflanzlichen Rohstoffen - einschliesslich Algen - beispielsweise Polysaccharide wie Pektine oder Stärke. Des Weiteren bevorzugt sind biotechnologisch hergestellte Verdickungsmittel mithilfe von nicht genmodifizierten Organismen (non GMO), wie beispielsweise Xanthan. Bevorzugt sind zudem anorganische Verdickungsmittel.

### Schaum

Generell zeigen bei der erfindungsgemässen Verwendung die Zubereitungen in Wasch- und Reinigungsanwendungen eine geringere Schaumbildung als herkömmliche Mittel auf Basis kurzkettiger Alkyltenside, welche typischerweise C8-C16 betragen, mit C12 als im Markt am häufigsten anzutreffender Alkylkettenlänge.

Es können bei der erfindungsgemässen Verwendung den Zubereitungen zusätzlich Schaumbildner zugegeben werden, insbesondere für Ausführungsformen, für die eine stärkere Schaumbildung erwünscht ist, als mit langkettigen Tensidmischungen üblicherweise erreicht wird. Die Schaumbildung bei Dusch- und Waschzubereitungen wird durch die Wahl der Tenside beeinflusst. Vorzugsweise werden für Ausführungsformen mit gewünschter Schaumbildung Kombinationen mit stark schäumenden Tensiden, beispielsweise Aminosäurederivate von Fettsäuren, wie Glycinate, Alanate, Taurate, oder Sarcosinate verwendet. Geeignet für eine gute Schaumbildung sind zudem amphotere Tenside, wie zum Beispiel Betaine, Imidazolcarboxylate oder Alkenylaminopropionsäuren.

In einer weiteren bevorzugten Ausführungsform der erfindungsgemässen Verwendung enthält die kosmetische Zubereitung Saponine als Schaumbildner. Saponine sind natürliche Glykoside, die als Pflanzeninhaltsstoffe weit verbreitet sind. Geeignet sind beispielsweise Saponine aus der indischen Waschnuss (Sapindus mukorossi), Koreanischen Ginsengs (Panax ginseng), Agavengewächsen, Inka-Gurke (Cyclanthera pedata), Süssholz (Glycyrrhiza glabra) und Seifenrinde (Quillaja saponaria Molina) und Mischungen derselben.

Besonders bevorzugt werden Saponine, die in Pflanzen der gemässigten Zone verbreitet sind, in der erfindungsgemässen Zubereitung eingesetzt. Hierbei handelt es sich um Saponine aus folgenden bevorzugten Pflanzen: Efeu (Hedera), Schlüsselblume (Primula veris), Vogelmiere (Stellaria media), Wald-Sanickel (Sanicula europaea), Dornige Hauhechel (Ononis spinosa), Hülsenfrüchten (Leguminosae), Spinat (Spinacia), Spargel (Asparagaceae), Hafer (Avena), (Ononis spinosa), Schattenblümchen (Maianthemum bifolium), Seifenkraut (Saponaria officinalis), Walnuss (Aesculus hippocastanum), Acker-Gauchheil (Anagallis arvensis), Gelber Hohlzahn (Galeopsis segetum), Karthäuser-Nelke (Dianthus carthusianorum), Ackerschachtelhalm (Equisetum arvense) und Mischungen derselben. Die Menge an Saponinen beträgt üblicherweise bis zu 5 Gew.-%, vorzugsweise 0,001 bis 3 Gew.-%, insbesondere 0,01 bis 2 Gew.-%.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Ethoxylierte Analoga von Alkyl- und/oder Alkenyloligoglykoside mit 8 bis 24 Kohlenstoffatomen im Alk(en)ylrest ;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Addukte mit 1 bis 30 Mol Ethylenoxid von Partialestern von Glycerin und/oder Sorbitan mit ungesättigten, linearen oder gesättigten, verzweigten Fettsäuren mit 12 bis 24 Kohlenstoffatomen und/oder Hydroxycarbonsäuren mit 3 bis 18 Kohlenstoffatomen
- Polyethylenglycol (Molekulargewicht 400 bis 5000)
- Alkoxylierte Partialester von Polyglycerin (durchschnittlicher Eigenkondensationsgrad 2 bis 8), sowie Partialester von Trimethylolpropan, Pentaerythrit, mit gesättigten und/oder ungesättigten, linearen oder verzweigten Fettsäuren mit 12 bis 24 Kohlenstoffatomen und/oder Hydroxycarbonsäuren mit 3 bis 18 Kohlenstoffatomen
- Mono-, Di- und Trialkylphosphate sowie Mono-, Di- und/oder Tri-PEGalkylphosphate und deren Salze;
- Polysiloxan-Polyalkyl-Polyether-Copolymere bzw. entsprechende Derivate;
- Block-Copolymere z. B. Polyethylenglycol-30 Dipolyhydroxystearate;
- Polymeremulgatoren, z. B. Pemulen-Typen
- Polyalkylenglycole
- Wollwachsalkohole
- Gemische derselben

Erfindungsgemäss bevorzugt werden folgende Emulgatoren, welche vorzugsweise auf Pflanzen der gemässigten Zonen beruhen:
- Partialester von Polyglycerin (durchschnittlicher Eigenkondensationsgrad 2 bis 8), Pentaerythrit, Zuckeralkoholen (z. B. Sorbit), Alkylglucosiden sowie Polyglucosiden (z. B. Cellulose) mit gesättigten und/oder ungesättigten, linearen oder verzweigten Fettsäuren mit 12 bis 24 Kohlenstoffatomen und/oder Hydroxycarbonsäuren mit 3 bis 18 Kohlenstoffatomen; insbesondere bevorzugt mit ungesättigten Fettsäuren von > 18 Kohlenstoffatomen.
- Polyglycerinester wie zum Beispiel Polyglyceryl-2 Dipolyhydroxystearate, Polyglycerin-3-Diisostearate, Polyglyceryl-4 Isostearate, Polyglyceryl-3 Oleate, Polyglyceryl-10-oleate, Polyglyceryl-10-dioleate, Diisostearoyl Polyglyceryl-3 Diisostearate, Polyglyceryl-3 Methylglucose Distearate, Polyglyceryl-3 Beeswax Polyglyceryl-4 Oleate, Polyglyceryl-3 Distearate und Polyglyceryl Polyricinoleate, Polyglyceryl Dimerate Isostearate sowie deren Gemische. Beispiele für weitere geeignete Polyolester sind umgesetzten Mono-, Di- und Triester von Trimethylolpropan oder Pentaerythrit mit nativen pflanzlichen Fettsäuregemischen aus Pflanzen der gemässigten Zone, sowie gereinigten pflanzlichen Fettsäuren wie Stearinsäure, Ölsäure, Behensäure und dergleichen.
- Alkyl- und/oder Alkenyloligoglykoside mit 8 bis 24 Kohlenstoffatomen im Alk(en)ylrest,
   insbesondere bevorzugt mit ungesättigten Fettsäuren von > 18
   Kohlenstoffatomen.
- Partialestern von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen.
   Insbesondere bevorzugt mit ungesättigten Fettsäuren von > 18 Kohlenstoffatomen, wie beispielsweise Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid,
   Des Weiteren besonders bevorzugt sind Glyceride basierend auf Pflanzen der gemässigten Zonen, wie beispielsweise Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozess noch geringe Mengen an Triglycerid enthalten können.
   Beispiele für bevorzugte Sorbitanester, welche auf Basis von Pflanzen der gemässigten Zonen hergestellt werden können, sind Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische.
- Mischester aus Pentaerythrit, Fettsäuren, Citronensäure und Fettalkohol und/oder Mischester von Fettsäuren mit 6 bis 24 Kohlenstoffatomen, wobei die Fettsäuren bzw. Fettalkohole auf Pflanzenölen der gemässigten Zonen beruhen, Methylglucose und Polyolen, vorzugsweise Glycerin oder Polyglycerin.
- Glycerincarbonat.
- Gemische derselben

### Anionische Emulgatoren

Typische anionische Emulgatoren sind aliphatische Fettsäuren mit 12 bis 24 Kohlenstoffatomen, wie beispielsweise Palmitinsäure, Stearinsäure oder Behensäure, sowie Dicarbonsäuren mit 12 bis 24 Kohlenstoffatomen, wie beispielsweise Azelainsäure oder Sebacinsäure.

Erfindungsgemäss bevorzugt sind Fettsäuren isoliert aus Pflanzen der gemässigten Zonen mit Kettenlängen > 18 C-Atomen wie Stearinsäure, Arachinsäure und Behensäure.

### Amphotere und kationische Emulgatoren

Weiterhin können als Emulgatoren zwitterionische Tenside, sowie kationische Emulgatoren verwendet werden, deren Strukturen in der vorliegenden Anmeldung im Abschnitt Tenside detailliert beschrieben wurden.

### Ölkörper

Erfindungsgemäss geeignet sind Ölkörper auf Basis von Fettalkoholen mit 6 bis 24, vorzugsweise 18 bis 24 Kohlenstoffatomen, Ester von linearen C6-C24-Fettsäuren mit linearen oder verzweigten C6-C24-Fettalkoholen bzw. Ester von verzweigten C6-C13-Carbonsäuren mit linearen oder verzweigten C6-C24-Fettalkoholen. Erfindungsgemäss bevorzugt sind Derivate mit Kettenlängen von C>18 und vorwiegend ungesättigt.

Geeignete Ester sind z.B. Myristylstearat, Myristylisostearat, Myristyloleat, Myristylbehenat, Myristylerucat, Cetylstearat, Cetylisostearat, Cetyloleat, Cetylbehenat, Cetylerucat, Stearylmyristat, Isostearylmyristat, Stearylpalmitat, Stearylstearat, Stearylisostearat, Isostearylpalmitat, Isostearylstearat, Isostearylisostearat, Oleylmyristat, Oleylpalmitat, Behenylmyristat Behenylpalmitat, Linolylmyristat, Linolylpalmitat, Linoleylmyristat, Linoleylpalmitat.
Bevorzugt werden bei der erfindungsgemässen Verwendung in den Zubereitungen Ester mit Kohlenwasserstoff (Alkyl)- und Acylresten mit C>18 eingesetzt, wie Derivate der der Stearinsäure, Behensäure, Erucasäure, Ölsäure, Linolsäure alpha-, sowie gamma-Linolensäure, zum Beispiel Stearyloleat, Stearylbehenat, Stearylerucat, Isostearyloleat, Isostearylbehenat, Isostearyloleat, Oleylstearat, Oleylisostearat, Oleylbehenat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Linoylstearat, Linoleylstearat.
Bei der erfindungsgemässen Verwendung besonders bevorzugt sind Ester mit ungesättigten Kohlenwasserstoff (Alkyl)- und Acylresten mit C>18, wie Derivate der Ölsäure, Linolsäure, alpha-, sowie gamma-Linolensäure, Erucasäure wie beispielsweise Oleyloleat, Oleyllinolat, Oleyllinolenat, Oleylrizinat, Oleylerucat, Linolyloleat Linolyllinolat, Linolylrizinat, Linolylerucat, Linolenyloleat, Linolenyllinolat, Linolenyllinenolat, Linolenylrizinat, Linolenylerucat,
Erucyloleat, Erucyllinolat, Erucyllinolenat, Erucylrizinat, Erucylerucat.
Wobei die Linolenderivate alpha-, sowie gamma- Linolenderivate einschliessen.

Daneben eignen sich weitere Ester wie sie vom Fachmann üblicherweise in kosmetischen Formulierungen eingesetzt werden, wie zum Beispiel lineare und verzweigte C6-C24-Fettalkoholcarbonate, Guerbetcarbonate auf Basis von Fettalkoholen mit 6 bis 24, vorzugsweise 18 bis 24 C-Atomen und ungesättigt, Ester der Benzoesäure mit linearen und/oder verzweigten C6-C24-Alkoholen, lineare oder verzweigte, symmetrische oder unsymmetrische Dialkylether mit 6 bis 24 Kohlenstoffatomen pro Alkylgruppe, Ringöffnungsprodukte von epoxidierten Fettsäureestern mit Polyolen, Siliconöle (Cyclomethicone, Siliciummethicontypen u. a.) und/oder aliphatische bzw. naphthenische Kohlenwasserstoffe, wie z. B. wie Squalan, Squalen oder Dialkylcyclohexane.
Vorzugsweise werden Ester eingesetzt, die auf Rohstoffe von Pflanzen der gemässigten Zone basieren, wie beispielsweise Pflanzenöle und entsprechende Mono-/Di- /Triglyceridmischungen auf Basis von C6-C24-Fettsäuren, Ester von C6-C24-Fettalkoholen und/oder Fettalkoholen mit Carbonsäuren oder Polyolen, wie zum Beispiel Zucker mit 2 bis 10 Kohlenstoffatomen und 2 bis 6 Hydroxylgruppen. In einer bevorzugten Ausführungsform werden keine Silikone eingesetzt.

### Öle, Fette und Wachse

Typische Beispiele für Fette und Öle sind Glyceride, d. h. feste oder flüssige pflanzliche oder tierische Produkte, die im Wesentlichen aus gemischten Glycerinestern höherer Fettsäuren bestehen, als Wachse kommen u. a. natürliche Wachse, wie z. B. Bienenwachs, Bürzelfett, Candelillawachs, Carnaubawachs, Ceresin, Espartograswachs, Guarumawachs, Japanwachs, Korkwachs, Lanolin (Wollwachs), Mikrowachse, Montanwachs, Ozokerit (Erdwachs), Petrolatum, Paraffinwachse, Ouricurywachs, Reiskeimölwachs, Schellackwachs, Walrat, Zuckerrohrwachs, chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt. Daneben kommen auch Sphingosine bzw. Sphingolipide in Frage. Desweiteren sind auch Mischungen der genannten Öle und Wachse möglich.

Bei der erfindungsgemässen Verwendung besonders bevorzugt eingesetzt werden Pflanzenöle, bzw. Triglyceride, wie sie aus den Pflanzen der gemässigten Zonen isoliert werden können. Ganz besonders bevorzugt werden Öle der Pflanzen und Pflanzenfamilien wie sie in der Anmeldung im Abschnitt "Pflanzen der gemässigten Zone" beschrieben wurden.

### Perlglanzwachse

Als Perlglanzwachse kommen beispielsweise in Frage: Alkylenglycolester, speziell Ethylenglycoldistearat; Fettsäurealkanolamide, Partialglyceride, Ester von mehrwertigen, gegebenenfalls hydroxysubstituierte Carbonsäuren mit Fettalkoholen mit 6 bis 24 Kohlenstoffatomen, Fettstoffe, wie beispielsweise Fettalkohole, Fettketone, Fettaldehyde, Fettether und Fettcarbonate, die in Summe mindestens 24 Kohlenstoffatome aufweisen; Fettsäuren wie Stearinsäure, Hydroxystearinsäure oder Behensäure, Ringöffnungsprodukte von Olefinepoxiden mit 12 bis 24 Kohlenstoffatomen mit Fettalkoholen mit 12 bis 24 Kohlenstoffatomen und/oder Polyolen mit 2 bis 15 Kohlenstoffatomen und 2 bis 10 Hydroxylgruppen sowie deren Mischungen.

Bei der erfindungsgemässen Verwendung bevorzugt sind Perlglanzwachse mit Kettenlängen von C>18, welche auf Basis von Pflanzenölen der gemässigten Zonen beruhen.

### Weitere Inhaltsstoffe

Neben den bisher genannten Komponenten können bei der erfindungsgemässen Verwendung die Zubereitungen weitere übliche Inhaltsstoffe für Kosmetika enthalten, die dem Fachmann durchaus bekannt sind.
Bei der erfindungsgemässen Verwendung können die kosmetischen und dermatologischen Zubereitungen dementsprechend ferner kosmetische Hilfsstoffe enthalten, wie sie üblicherweise in solchen Zubereitungen verwendet werden; beispielsweise Konsistenzgeber, Konservierungsmittel, Stabilisatoren, Füllstoffe, Parfüme, Pigmente, die färbende Wirkung haben, Verdickungsmittel, Konsistenzmittel, Suspendiermittel, Puffergemische, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Wasser, Salze, antimikrobiell, proteolytische oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte, sowie Gemische derselben.

Bei der erfindungsgemässen Verwendung können die Zubereitungen als weitere Hilfs- und Zusatzstoffe weitere Ölkörper, Emulgatoren und Co-Emulgatoren, sowie im kosmetischen, pharmazeutischen und dermatologischen Bereich gebräuchliche Zusätze, wie z.B. kationische Polymere, Filmbildner, Überfettungsmittel, Stabilisatoren, biogene Wirkstoffe, Lösungsmittel, Trübungsmittel, ferner Eiweissderivate wie Gelatine, Collagenhydrolysate, Aminosäuren, Oligo- oder Polypeptide auf natürlicher und synthetischer Basis, Eigelb, Lanolin und Lanolinderivate, deodorierende Mittel, Stoffe mit keratolytischer und keratoplastischer Wirkung, Enzyme und Trägersubstanzen, Pflanzenextrakte, Vitamine und Antiageing- Mittel enthalten. Des Weiteren können den erfindungsgemäss verwendeten Mitteln antimikrobiell wirkende Agentien zugesetzt werden.

Bei der erfindungsgemässen Verwendung können die Zubereitungen in Form von Gelen oder Lotionen vorliegen. So können sie z. B. eine Lösung, eine Emulsion (O/W), oder eine multiple Emulsion, beispielsweise vom Typ Wasser-in-ÖI-in-Wasser (W/O/W), ein Gel, eine Hydrodispersion, eine lamellare Phase, eine flüssige isotrope Lösungsphase oder eine micellare Phase, darstellen.

Bevorzugte Ausführungsformen der Zubereitungen werden angewendet als Duschzubereitungen, Badezusätze, Handseifen, Intimreinigung, Gesichtstonic, Reinigung des Augenbereichs, Gele, Lotionen, Rasierprodukte, Haarshampoos, konditionierende Shampoos, Haar-Tonics, Haarfärbeshampoos, Haarfärbemittel und Haarstyling-Zubereitungen.

Bei der erfindungsgemässen Verwendung handelt es sich bei den Zubereitungen um Rinse-off-Formulierungen, insbesondere um Shampoos, Duschbäder, Duschgels, Schaumbäder und Flüssigseifen. Moderne Rinse-off Produkte haben häufig einen hohen Anteil an konditionierenden Wirkstoffen, die auch aus Ölanteilen bestehen können. Folglich können diese Zubereitungen als Emulsionen vorliegen.

Bevorzugt können die kosmetischen Zubereitungen als milde Reinigungsmittel für sensible Haut, für Konsumenten mit Allergien, für Kinder- und Babyprodukte und/oder für Haushalte mit Kindern verwendet werden.

In einer bevorzugten Ausführungsform können die kosmetischen Zubereitungen in der Tierkosmetik verwendet werden.

### AUSFÜHRUNGSBEISPIELE

Es wurden für die erfindungsgemässe Verwendung Pflege- und Reinigungsmittel hergestellt, beispielhafte Zusammensetzungen sind der Tabelle zu entnehmen. Die Mengenangaben sind dabei in Gew.-% Aktivstoff.

**Tabelle 1:**

| Beispiele von Zusammensetzungen von Reinigungsmitteln für Körper und Haar für die erfindungsgemässe Verwendung | | | | | | | |
|---|---|---|---|---|---|---|---|
| **Referenz- Beispiel 1** | | **Beispiel 2** | | **Referenz- Beispiel 3** | | **Beispiel 4** | |
| **z.B. Handseife** | | **z.B. Shampoo** | | **z.B. Duschzubereitung** | | **z.B. Gesichtsreinigung** | |
| **Inhaltsstoffe** | **Gew.%** | **Inhaltsstoffe** | **Gew.%** | **Inhaltsstoffe** | **Gew.%** | **Inhaltsstoffe** | **Gew.%** |
| Aqua | 91.8 | Aqua | 92.0 | Aqua | 83.3 | Aqua | 95.0 |
| Hazelnut fatty acids, potassium salts | 4.5 | Olivamido propyl betaine | 4.5 | Sodium methyl oleyl taurate | 10.0 | Canolamido propyl betaine | 3.0 |
| Disodium oleamido MEA-sulfosuccinate | 3.0 | Sodium grapeseed amphoacetate | 3.0 | Oleamidopropyl hydroxysultaine | 4.0 | Olive oil polyglyceryl-6-ester | 1.0 |
| Glycerin | 0.3 | Glycerin | 0.3 | Sodium oleyl methyl isethionate | 2.0 | Senum indicum PEG-8 ester | 1.0 |
| Dipotassium Glycyrrhizate | 0.2 | Dipotassium Glycyrrhizate | 0.2 | Hydrolyzsed wheat protein | 0.2 | Aktivstoffe, Verdicker, Farbstoffe, Parfüm, Konservierungsmittel | q.s. |
| | | | | Aesculus Hippocastanum extract | 0.5 | | |
| Aktivstoffe, Verdicker, Farbstoffe, Parfüm, Konservierungsmittel | q.s. | Aktivstoffe, Verdicker, Farbstoffe, Parfüm, Konservierungsmittel | q.s. | Aktivstoffe, Verdicker, Farbstoffe, Parfüm, Konservierungsmittel | q.s. | | |

Die für die Erfindung verwendeten Seifen wie beispielsweise Hazelnut fatty acids, potassium salt wurden nach üblichen, literaturbekannten Verfahren aus Fettsäuren-, deren Gemischen oder Pflanzenölen hergestellt.

Oleamido propyl hydroxysultaine und Canolamido propyl betaine wurde analog literaturbekannten Verfahren für Cocoamido Derivate hergestellt.

Andere Rohstoffe sind kommerziell erhältlich, wie zum Beispiel Disodium oleamido MEA-sulfosuccinate von Rhodia oder Colonial, Sodium grapeseed amphoacetate von RES Pharma, Oliveamido propyl betaine von Soliance, Dipotassium Glycyrrhizate von Nikkol oder R.I.T.A. Corporation, Sodium oleyl methyl isethionate, Hydrolyzed Wheat protein von Cognis/BASF, Aesculus Hippocastanum extract von Indena, Olive oil polyglyceryl-6-ester und Senum indicum PEG-8 ester von Res Pharma.

## Patentansprüche

1. Verwendung von wässrigen, flüssigen Tensidmischungen als kosmetische Pflege- oder Reinigungszubereitungen in Form von Rinse-off-Formulierungen für Körper und Haare, wobei ein oder mehrere Tenside aus den Klassen der anionischen, nichtionischen, zwitterionischen und amphoteren Tensiden ausgewählt sind aus der Gruppe umfassend:
a) Fettsäuren und deren Alkali oder Ammoniumsalze mit den allgemeinen Formeln R(O)OH bzw. R(O)OM mit M = Alkalimetallkation oder Ammonium
b) Fettsäureester mit der allgemeinen Formel R(O)OR',
c) Ether von Fettalkoholen mit der allgemeinen Formel ROR',
d) Fettsäureamide mit der allgemeinen Formel R(O)NR',
e) Fettsäureimine der allgemeinen Formel R(NR')NR"R"', bevorzugt Amphoacetate
f) Primäre, sekundäre, tertiäre oder quartäre Fettamine bzw. Fettammoniumsalze der allgemeinen Formeln RNH2, RHNR', RNR'R" und (R)(R')(R")(R"')N+X-,
g) sowie Gemischen derselben
wobei
i) R' bis R'" organische oder anorganische hydrophile Reste darstellen und X- für ein Anion steht
ii) R den lipophilen Teil des Tensids darstellt und aus einem linearen, gesättigten oder ungesättigten, unverzweigten Kohlenwasserstoffrest mit 8 bis 24 Kohlenstoffatomen besteht;
und der Anteil an Kohlenwasserstoffresten R von 18 und mehr C-Atomen über 60 Gew.-%, vorzugsweise über 70 Gew.-% und besonders bevorzugt zwischen 80-100 Gew.-% beträgt
und dass gleichzeitig der Anteil an einfach oder mehrfach ungesättigten Kohlenwasserstoffresten R zwischen 50-100 Gew.-%, vorzugsweise zwischen 60-100 Gew.-% und besonders bevorzugt zwischen 70-90 Gew.-% beträgt, jeweils bezogen auf den Gesamtgehalt an Kohlenwasserstoffreste R der in der Zubereitung enthaltenen Tenside;
und wobei der Anteil an Tensiden auf Basis von Pflanzenölen, -wachsen, -fetten oder -harzen 80-100 Gew.-%, bezogen auf den Gesamtgehalt an Tensiden in der Zubereitung, beträgt und ausgewählt ist aus der Gruppe umfassend: Amarant, Anis, Apfel, Aprikose, Arnika, Avocado, Baumwolle, Borretsch, Brokkoli, Hanf, Haselnuss, Buche, Buchsbaum, Distel, Dinkel, Erdnuss, Erdmandel, Flieder, Gartenkresse, Gerste, Granatapfel, Hafer, Heidelbeere, Holunder, Jasmin, Jatropha, Johannisbeere, Johanniskraut, Jojoba, Kamelie, Kamille, Kümmel, Karotte, Kirsche, Koriander, Königskerze, Krambe, Kreuzblättrige Wolfsmilch, Kürbis, Iberischer Drachenkopf, Lavendel, Leindotter, Leinsamen, Liguster, Lupine, Luzerne, Mais, Mandel, Mirabelle, Mango, Mohn, Nachtkerze, Olive, Ölrettich, Ölrauke, Pekannuss, Pfirsich, Pflaume, Pistazie, Preiselbeere, Raps, Reis, Ringelblume, Rübsen, Saflor, Salbei, Sanddorn, Schwarzkümmel, Sesam, Sesamblatt, Senf, Sonnenblume, Soja, Tabak, Walnuss, Weintraube, Weizen, Wiesenschaumkraut und Wildrose, sowie deren Kombinationen;
und mindestens ein Tensid ausgewählt aus a) bis g) enthalten ist, das keine Seife ist.

2. Verwendung nach Anspruch 1 wobei das oder die Tenside a) bis g) ausgewählt sind aus der Gruppe umfassend: Seifen aus Alkali- oder Ammoniumsalz(e) von Fettsäuren (Seifen), Alkylbenzolsulfonate, Alkan-/Alkensulfonate, Alkylsulfate, Alkylethersulfate, Fettalkoholsulfate, Sulfosuccinate, Sulfosuccinatester, Sulfobernsteinsäuremono- und di-Alkylester, α-Olefinsulfonate, Alkylsulfoacetate, sulfonierte Fettsäuren, sulfonierte Fettsäureester, sulfonierte Fettsäureglycerinester, sulfonierte Fettsäuremethylester, Alkylphosphate, Alkyletherphosphate, Phosphor- und Polyphosphorsäureester, Carbonsäureamidethersulfate, N-Acylaminosäurederivate, N-Acylaspartat, N-Acylglycinat, N-Acylalaninat, N-Acylsarkosinat, N-Acylglutamat, acylierte Polypeptide, N-Acylaminosulfonsäuren, N-Acyltaurid, Alkylisethionat, Acylisethionat, Acyllactylat, Fettalkoholcarboxylat, Alkylpolyglykolethercarboxylat, Alkoholpolyglykolether und Carbonsäurepolyglykolester, ethoxylierte und propoxylierte Fettalkohole, ethoxylierte und/oder propoxylierte Alkohole oder Carbonsäuren mit 1-40 Ethylenoxid (EO) und/oder Propylenoxid (PO)- Einheiten, Aminoxide, Polyethylenglykolmercaptane, Glykolipide, Alkylpolyglykoside mit 1-10 Glykosideinheiten, Polyhydroxyfettsäureamide, Polyoxyethylenglycole, Polyhydroxyfettsäureester, Glyceryl- und Polyglycerylester mit 1-20 Glycerineinheiten, Glycolipide, Alkylpolyglycoside mit 1-10 Glycosideinheiten, Carbonsäureester, Sorbitanester, alkoxylierte Sorbitanester, Alkanolamin-Carbonsäure-Kondensate, N-Alkylpyrrolidone, Amidoalkylpyrrolidone, N-Alkylaminopropionsäuren, Alkyl-iminodipropionsäuren, Imidazolcarboxylate, Amphoacetate, N-Alkylbetaine, Aminobetaine, acylierte Diamine und Polyamine, Amidoamine und Amidobetaine, Aminoxide, Sulfobetaine, Sultaine, Acylisethionate, Polyoxyethylen (POE) amine, Imidazolinium ammonium ester quats, quaternisierte POE Amine, sowie deren Salze.

3. Verwendung gemäss einem der vorstehenden Ansprüche, **dadurch gekennzeichnet, dass** eines oder mehrere Tenside verwendet werden, deren Verteilung an Kohlenwasserstoffresten unterschiedlicher Kettenlänge und Sättigungsgrad dem Vorkommen im natürlichen Öl entsprechen und bis zu 15 Gew.-%, vorzugsweise bis zu 13 Gew.-%, und besonders bevorzugt zwischen 0.1 und 11 Gew.-% an Glyzerin enthält; Gew.-% Aktivgehalt bezogen auf den Gesamtgehalt an Tensiden.

4. Verwendung gemäss einem der vorstehenden Ansprüche, wobei der Anteil an kurzen Kohlenwasserstoffresten R in den Tensiden mit Kettenlängen <=C16 unter 20 Gew.-%, bevorzugt unter 15 Gew.-%, und insbesondere <= C 14 unter 5 Gew.- % beträgt, jeweils bezogen auf den Gesamtgehalt an Kohlenwasserstoffresten R der in der Zubereitung enthaltenen Tenside.

5. Verwendung gemäss einem der vorstehenden Ansprüche, wobei der Anteil an Tensiden auf Basis von Pflanzenölen, -fetten, -wachsen oder -harzen der gemässigten Zone vorzugsweise mehr als 95 Gew.-% und besonders bevorzugt 99-100 Gew.-% beträgt jeweils bezogen auf den Gesamtgehalt an Tensiden.

6. Verwendung gemäss einem der vorstehenden Ansprüche, wobei eines oder mehrere Tenside verwendet werden, welche vorzugsweise abgeleitet sind von einem Pflanzenöl, -wachs, -fett oder -harz ausgewählt aus der Gruppe: Brokkoli, Hanf, Haselnuss, Buche, Distel, Dinkel, Erdmandel, Gerste, Kirsche, Königskerzen, Krambe, Kreuzblättrige Wolfsmilch, Kürbis, Iberischer Drachenkopf, Leindotter, Leinsamen, Lupine, Luzerne, Mais, Mohn, Nachtkerze, Olive, Ölrettich, Ölrauke, Raps, Reis, Ringelblume, Rübsen, Saflor, Sanddorn, Schwarzkümmel, Salbei, Sesam, Sesamblatt, Senf, Sonnenblume, Soja, Tabak, Walnuss, Weintraube und Weizen, sowie Gemischen derselben, ganz besonders bevorzugt aus der Gruppe: Distel, Erdmandel, Krambe, Iberischer Drachenkopf, Leindotter, Leinsamen, Lupine, Luzerne, Olive, Ölrettich, Ölrauke, Raps, Rübsen, Sesamblatt, Sonnenblume, Soja, Weintraube und Weizen, sowie Gemischen derselben.

7. Verwendung gemäss einem der vorstehenden Ansprüche, **dadurch gekennzeichnet, dass** die Pflege- oder Reinigungszubereitung für Körper und Haare zusätzlich einen oder mehrere kosmetische Inhaltstoffe enthält, ausgewählt aus den Gruppen umfassend:
a) Antioxidantien
b) Saponine
c) konditionierende Mittel
d) UV Filter
d) Öle, Fette und Wachse
e) Ölkörper

8. Verwendung der Zubereitung gemäss einem der vorstehenden Ansprüche als milde Reinigungsmittel zur Anwendung für sensible Haut, für Konsumenten mit Allergien, Anwendung in Kinder- und Babyprodukten und/oder Haushalten mit Kindern.

## Claims

1. Use of aqueous, liquid surfactant mixtures as cosmetic care or cleaning preparations in the form of rinse-off formulations for body and hair, whereas one or more surfactants are selected from the classes of anionic, nonionic, zwitterionic and amphoteric surfactants from the group comprising:
a) fatty acids and their alkali or ammonium salts with the general formulae R(O)OH and R(O)OM with M = alkali metal cation or ammonium,
b) fatty acid esters having the general formula R(O)OR',
c) ethers of fatty alcohols having the general formula ROR',
d) fatty acid amides having the general formula R(O)NR',
e) fatty acid imines having the general formula R(NR')NR"R"', preferably amphoacetates,
f) primary, secondary, tertiary or quaternary fatty amines or fatty ammonium salts having the general formulae RNH₂, RNHR', RNR'R" and (R)(R')(R")(R"')N⁺X⁻,
g) and mixtures thereof
whereby
i) R' to R'" represent organic or inorganic hydrophilic moieties and X⁻ represents an anion;
ii) R represents the lipophilic moiety of the surfactant and consists of a linear, saturated or unsaturated, unbranched hydrocarbon radical having 8 to 24 carbon atoms;
and the proportion of hydrocarbon radicals R of 18 and more C-atoms is above 60 wt.-%, preferably above 70 wt.-% and particularly preferably between 80 - 100 wt.-% and simultaneously the proportion of monounsaturated or polyunsaturated hydrocarbon radicals R is between 50 and 100 wt.-%, preferably between 60 - 100 wt-.% and particularly preferably between 70-90 wt.-%,
in each case based on the total content of hydrocarbon radicals R of surfactants present in the preparation;
and wherein the proportion of surfactants based on vegetable oils, vegetable waxes, vegetable fats or vegetable resins is 80-100 wt.-%, based on the total amount of surfactants in the preparation, selected from the group comprising: amaranth, anise, apple, apricot, arnica, avocado, cotton, borage, broccoli, hemp, hazelnut, beech, boxwood, thistle, spelt, peanut, yellow nutsedge, lilac, garden cress, barley, pomegranate, oats, vaccinium, elder, jasmine, jatropha, currant, St. John's wort, jojoba, camellia, chamomile, caraway, carrot, cherry, coriander, mullein, crambe, caper spurge, squash, Iberian dragon head, lavender, camelina, linseed, privet, lupine, lucerne, corn, almond, mirabelle, mango, poppy, evening primrose, olive, oilseed radish, rocket, pecan, peach, plum, pistachio, cranberry, rapeseed, rice, marigold, turnip rape, safflower, sage, sea buckthorn, black cumin, sesame, sesame leaf, mustard, sunflower, soy, tobacco, walnut, grape, wheat, meadowfoam and wild rose, as well their combinations,
and wherein at least one surfactant selected from a) to g) is present, which is not a soap.

2. Use according to claim 1, wherein the surfactant or surfactants a) to g) are selected from the group comprising: Soaps from alkali or ammonium salt(s) of fatty acids (soaps), alkylbenzene sulfonates, alkane / alkene sulfonates, alkyl sulfates, alkyl ether sulfates, fatty alcohol sulfates, sulfosuccinates, esters of sulfosuccinates, sulfosuccinic acid mono- and dialkyl esters, α-olefin sulfonates, alkyl sulfoacetates, sulfonated fatty acids, sulfonated fatty acid esters, sulfonated fatty acid glycerol esters, sulfonated fatty acid methyl esters, alkyl phosphates, alkyl ether phosphates, phosphoric acid esters and poly phosphoric acid esters, carboxylic acid amide ether sulfates, N-acyl amino acid derivatives, N-acyl aspartate, N-acyl glycinate, N-acyl alaninate, N-acyl sarcosinate, N-acyl glutamate, acylated polypeptides, N-acylamino sulfonic acids, N-acyl tauride, alkyl isethionate, acyl isethionate, acyl lactylate, fatty alcohol carboxylate, alkyl polyglycolether carboxylate, alcohol polyglycolethers and carboxylic acid polyglycol esters, ethoxylated and propoxylated fatty alcohols, ethoxylated and/or propoxylated alcohols or carboxylic acids with 1-40 ethylen oxide (EO) and/or propylene oxide (PO) entities, amine oxides, polyethylene glycol mercaptanes, glycolipids, alkyl polyglycosides with 1-10 glycoside entities, polyhydroxy fatty acid amides, polyoxyethylene glycols, polyhydroxy fatty acid esters, glyceryl and polyglyceryl esters with 1-20 glycerine entities, glycolipids, alkyl polyglycosides with 1-10 glycoside entities, carboxylic acid esters, sorbitane esters, alkoxylated sorbitane esters, alkanolamine- carboxylic acid-condensates, N-alkyl pyrrolidones, amido alkyl pyrrolidones, N-alkyl amino propionic acids, alkyl imino dipropionic acids, imidazolecarboxylate, amphoacetates, N-alkyl betaines, amino betaines, acylated diamines and polyamines, amido amines and amido betaines, amine oxides, sulfo betaines, sultaines, acyl isethionates, polyoxyethylene (POE) amines, imidazolinium ammonium ester quats, quaternized POE amines, and the salts thereof.

3. Use according to one of the preceding claims, **characterized in that** one or more surfactants are used, whose distribution of hydrocarbon radicals of different chain lengths and degrees of saturation corresponds to the occurrence in natural oil and that up to 15 wt.-%, preferably up to 13 wt.-% and particularly preferred between 0.1 and 11 wt.-% of glycerine are present; wt.-% active content based on the total content of surfactants.

4. Use according to one of the preceding claims, **characterized in that** the proportion of short hydrocarbon radicals R in the surfactants with chain lengths of R ≤ C16 is below 20 wt.-%, preferably below 15 wt.-%, and in particular <= C 14 is less than 5 wt.-%, in each case based on the total content of hydrocarbon radicals R of the surfactants contained in the preparation.

5. Use according to one of the preceding claims, **characterized in that** the proportion of surfactants based on vegetable oils, vegetable fats, vegetable waxes or vegetable resins of the temperate zone is preferably more than 95 wt .-% and particularly preferably 99-100 wt.-%, in each case based on the total content of surfactants.

6. Use according to one of the preceding claims, **characterized in that** one or more surfactants are used, which preferably are based on a vegetable oil, vegetable wax, vegetable fat or vegetable resin selected from the group: broccoli, hemp, hazelnut, beech, thistle, spelt, yellow nutsedge, barley, cherry, mullein, crambe, caper spurge, squash, Iberian dragon head, camelina, linseed, lupine, lucerne, corn, poppy, evening primrose, olive, oilseed radish, rocket, rapeseed, rice, marigold, turnip rape, safflower, sea buckthorn, black cumin, sage, sesame, sesame leaf, mustard, sunflower, soy, tobacco, walnut, grape and wheat, as well their combinations, particularly preferably selected from the group: thistle, yellow nutsedge, crambe, Iberian dragon head, camelina, linseed, lupine, lucerne, olive, oilseed radish, rocket, rapeseed, turnip rape, sesame leaf, sunflower, soy, grape and wheat, as well their combinations.

7. Use according to one of the preceding claims, **characterized in that** the care or cleaning preparation for body and hair additionally contains one or more cosmetic ingredients, selected from the group:
a) antioxidants
b) saponins
c) conditioning agents
d) UV filters
e) oils, fats and waxes
f) emollients

8. Use of the preparation according to one of the preceding claims as mild cleansing agent for the application for sensitive skin, for consumers with allergies, application in products for children and babies and/or for households with children.

## Revendications

1. Utilisation de mélanges de tensioactifs aqueux et liquides comme préparations cosmétiques de soin ou de nettoyage sous forme de formulations à rincer pour le corps et les cheveux, dans lesquels un ou plusieurs tensioactifs des classes de tensioactifs anioniques, non ioniques, zwitterioniques et amphotères sont choisis dans le groupe comprenant:
a) les acides gras et leurs sels alcalins ou d'ammonium, répondant aux formules générales R(O)OH ou R(O)OM avec M = cation de métal alcalin ou ammonium,
b) esters d'acides gras répondant à la formule générale R(O)OR',
c) éthers d'alcools gras répondant à la formule générale ROR',
d) amides d'acides gras répondant à la formule générale R(O)NR',
e) imines d'acides gras répondant à la formule générale R(NR')NR"R"', de préférence des amphoacétates,
f) amines grasses primaires, secondaires, tertiaires ou quaternaires ou sels d'ammonium gras de formules générales RNH2, RNHR', RNR'R" and (R)(R')(R")(R"')N⁺X⁻,
g) et leurs mélanges
dans lesquels
i) R' à R'" représentent des résidus hydrophiles organiques ou inorganiques et X⁻ représente un anion;
ii) R représente la partie lipophile de l'agent tensioactif et consiste en un radicale hydrocarboné linéaire, saturé ou insaturé, non ramifié ayant 8 à 24 atomes de carbone;
et la proportion de radicaux hydrocarbonés R de 18 d'atomes de carbone et plus est supérieure à 60 % en poids, de préférence supérieure à 70% en poids et de manière particulièrement préférée comprise entre 80 et 100% en poids
et, dans le même temps, la proportion de radicaux hydrocarbonés mono- ou polyinsaturés R est comprise entre 50-100 % en poids, de préférence comprise entre 60- 100 % en poids et de manière particulièrement préférée comprise entre 70 et 90% en poids, chacun par rapport à la teneur totale en radicaux hydrocarbonés R des agents tensioactifs présents dans la préparation;
et dans lesquels la proportion des tensioactifs à base d'huiles végétales, de cires végétales, de grasses végétales ou de résines végétales est 80 à 100% en poids, par rapport à la teneur totale en agents tensioactifs présents dans la préparation et choisis dans le groupe comprenant: amarante, anis, pomme, abricot, arnica, avocat, coton, bourrache, brocoli, chanvre, noisette, hêtre, buis, chardon, épeautre, arachide, souchet comestible, lilas, cresson alénois, orge, grenade, avoine, bleuet, sureau, jasmin, jatropha, groseille, millepertuis, jojoba, camélia, camomille, carvi, carotte, cerise, coriandre, molène, crambe, euphorbe épurge, courge, tête de dragon ibérique, lavande, caméline, graines de lin, troène, lupin, luzerne, maïs, amande, mirabelle, mangue, pavot, onagre, olive, radis oléagineux, roquette, noix de pécan, pêche, prune, pistache, airelle, colza, riz, souci, navette, carthame, sauge, argousier, cumin noir, sésame, feuille de sésame, moutarde, tournesol, soja, tabac, noix, raisin, blé, limnanthe, et rose musquée, ainsi que leurs combinaisons;
et dans lesquels au moins un agent tensioactif choisi parmi a) à g) autre que du savon est présent.

2. Utilisation selon la revendication 1, dans laquelle le ou les agents tensioactifs a) à g) sont choisis dans le groupe comprenant: Savons de sels alcalins ou d'ammonium d'acides gras (savons), alkylbenzènesulfonates, sulfonates d'alcane/alcène, sulfates d'alkyle, sulfates d'éther alkylique, sulfates d'alcool gras, sulfosuccinates, esters sulfosucciniques, esters mono- et dialkyliques d'acide sulfosuccinique, α-oléfinesulfonates, alkyl sulfoacétates, acides gras sulfonés, esters d'acides gras sulfonés, esters de glycérol d'acides gras sulfonés, esters méthyliques d'acides gras sulfonés, phosphates d'alkyles, éthers d'alkyl phosphate, esters phosphoriques et polyphosphoriques, éthersulfates d'amide d'acide carboxylique, dérivés de N-acylaminoacides, N-acyl aspartate, N-acyl glycinate, N-acyl alaninate, N-acyl sarcosinate, N-acyl glutamate, polypeptides acylés, acides N-acyl aminosulfoniques, N-acyl tauride, alkyliséthionate, acyliséthionate, lactate d'acyle, carboxylate d'alcool gras, carboxylates d'alkyl polyglycol éther, alcool polyglycol éther et ester de polyglycole d'acide carboxylique, alcools gras éthoxylés et propoxylés, alcools ou acides carboxyliques éthoxylés et / ou propoxylés avec 1-40 unités d'oxyde d'éthylène (EO) et / ou d'oxyde de propylène (PO), oxydes d'amines, polyéthylène glycol mercaptans, glycolipides, alkylpolyglycosides avec 1 à 10 unités glycosidiques, amides d'acides gras polyhydroxylés, polyoxyéthylèneglycols, esters d'acides gras polyhydroxylés, esters glycériques et polyglycériques avec 1-20 unités de glycérines, glycolipides, alkyl polyglycosides avec 1 à 10 unités glycosidiques, esters d'acides carboxyliques, esters de sorbitan, esters de sorbitan alcoxylés, condensats d'alcanolamines - acides carboxyliques, N-alkyl pyrrolidones, amidoalkyl pyrrolidones, acides N-alkylamino propioniques, acides alkyl-iminodipropioniques, carboxylates d'imidazole, amphoacétates, N-alkylbétaines, aminobétaines, diamines et polyamines acylées, amidoamines et amidobétaines, oxydes d'amines, sulfobétaines, sultaines, acyliéthionates, polyoxyéthylène (POE) aminés, imidazolinium ammonium ester quats, amines POE quaternisées et leurs sels.

3. Utilisation selon l'une des revendications précédentes, **caractérisée en ce que** l'on utilise un ou plusieurs agents tensioactifs dont la répartition des radicaux hydrocarbonés de différentes longueurs de chaînes et degrés de saturation correspond à celle de l'huile naturelle et contient jusqu'à 15 % en poids, de préférence jusqu'à 13 % en poids, et de manière particulièrement préférée entre 0,1 et 11 % en poids de glycérol; teneur active % en poids sur la base de la teneur totale des agents tensioactifs.

4. Utilisation selon l'une des revendications précédentes, **caractérisée en ce que** la proportion de radicaux hydrocarbonés courts R dans les agents tensioactifs ayant des longueurs de chaines ≤ C16 est inférieure à 20 % en poids, de préférence inférieure à 15 % en poids et de manière particulièrement préférée <= C 14 est inférieure à 5% en poids; % en poids dans chaque cas par rapport à la teneur totale des radicaux hydrocarbonés R des agents tensioactifs contenus dans la préparation.

5. Utilisation selon l'une des revendications précédentes, **caractérisée en ce que** la proportion d'agents tensioactifs à base d'huiles végétales, de graisses végétales, de cires végétales ou de résines végétales de la zone tempérée est de préférence supérieure à 95% en poids et, de manière particulièrement préférée, 99 à 100% en poids; dans chaque cas par rapport à la teneur totale en agents tensioactifs.

6. Utilisation selon l'une des revendications précédentes dans laquelle on utilise un ou plusieurs agents tensioactifs qui sont de préférence dérivés d'une huile végétale, d'une cire végétale, d'une graisse végétale ou d'une résine végétale choisie dans le groupe: brocoli, chanvre, noisette, hêtre, chardon, épeautre, souchet comestible, orge, cerise, molène, crambe, euphorbe épurge, courge, tête de dragon ibérique, caméline, graines de lin, lupin, luzerne, maïs, pavot, onagre, olive, radis oléagineux, roquette, colza, riz, souci, navette, carthame, argousier, cumin noir, sage, sésame, feuille de sésame, moutarde, tournesol, soja, tabac, noix, raisin, blé, ainsi que leurs combinaisons.

7. Utilisation selon l'une des revendications précédentes, **caractérisée en ce que** la préparation de soin ou de nettoyage du corps et des cheveux contient en outre un ou plusieurs ingrédients cosmétiques choisis parmi les groupes comprenant:
a) les antioxydants
b) les saponines
c) les agents de conditionnement
d) les filtres UV
d) les huiles, graisses et cires.
e) les émollients

8. Utilisation de la préparation selon l'une des revendications précédentes comme nettoyant doux pour l'application sur les peaux sensibles, pour les consommateurs souffrant d'allergies, pour l'application dans les produits pour enfants et bébés et/ou pour les ménages avec enfants.
